# EUROPEAN PATENT SPECIFICATION

(11) **EP 2 403 641 B1**
(45) Date of publication and mention of the grant of the patent: **16.07.2014**
(21) Application number: 10707009.6
(22) Date of filing: 03.03.2010
(51) Int. Cl.: B01J 21/16, B01J 29/70, B01J 35/00, C01B 33/20, C01B 37/00, C01B 39/02, C07C 209/16

(54) **PROCESS FOR THE PREPARATION OF AN ISOMORPHOUSLY SUBSTITUTED SILICATE**
VERFAHREN ZUR HERSTELLUNG EINES ISOMORPH SUBSTITUIERTEN SILICATS
PROCÉDÉ DE PRÉPARATION D'UN SILICATE SUBSTITUÉ DE FAÇON ISOMORPHE

(30) Priority: 03.03.2009 WO PCT/CN2009/070620
(43) Date of publication of application: 11.01.2012
(73) Proprietor: BASF SE, 67056 Ludwigshafen (DE); Tokyo Institute of Technology, Tokyo 152-8550 (JP)
(72) Inventor: YILMAZ, Bilge, New York NY 10014 (DE); MÜLLER, Ulrich, 67435 Neustadt (DE); PFAFF, Meike, 66539 Neunkirchen (DE); XIAO, Feng-Shou, Changchun 130023 (CN); GIES, Hermann, 45549 Sprockhövel (DE); DE VOS, Dirk, B-3220 Holsbeek (BE); BAO, Xinhe, Dalian 116011 (CN); ZHANG, Weiping, 116023 Dalian (CN); TATSUMI, Takashi, Kawasaki 215-0021 (JP)
(74) Representative: Altmann, Andreas
(86) International application number: PCT/EP2010/052713
(87) International publication number: WO 2010/100203

(56) References cited:
- WO-A1-02/22502
- WO-A2-2008/122579
- US-A- 6 063 944
- US-A1- 2005 065 016
- US-A1- 2008 253 953
- SONG J ET AL: "Zeolites synthesis in the system N(CH3)(C2H5)3F-SiO2-H2O" STUDIES IN SURFACE SCIENCE AND CATALYSIS, ELSEVIER BV, NL LNKD- DOI:10.1016/S0167-2991(04)80815-2, vol. 154, no. 1, 1 January 2004 (2004-01-01), pages 295-300, XP008126972 ISSN: 0167-2991 [retrieved on 2007-08-28]

## Description

The present invention relates to a process for the preparation of an isomorphously substituted silicate. Further, the present invention relates to an isomorphously substituted silicate obtainable or obtained by this process. The present invention also encompasses a further step in the manufacturing process, namely the step of preparing isomorphously substituted tectosilicates from the layered silicate Additionally, the present invention also relates to the use of said materials.

In various technical areas, such as, for example, catalysis or adsorption, there is a constant need for new materials, in particular silicates, and new processes, respectively, allowing for manufacturing tailor-made materials for, e.g., specific catalytic or adsorption problems.

The synthesis of, for example, all-silica silicates or substituted silicates of structure type RUB-39, are disclosed in WO 2005/100242, WO 2007/042531, or WO2008/122579.

US 2008/253953 relates to a one-step synthetic methodology for the preparation of RUB-39 and RUB-41 silicates which may further include aluminum in their framework structure.

WO 02/22502 concerns a one-step synthetic methodology for the preparation of an isomorphously substituted zeolite by heteroatom substitution in large and extra-large pore borosilicate zeolites.

Finally, Song et al. in Studies in Surface Science and Catalysis 2004, 154, 295-300 relates to the crystallization of microporous materials including RUB-36.

In particular for industrial applications, there is a need for providing efficient processes for the preparation of these materials. Moreover, there is constant need for processes which allow for obtaining products having better purity and better crystallinity. Additionally, also higher yields are of importance for industrial purposes.

Therefore, it is an object of the present invention to provide a new process for the preparation of isomorphously substituted layered silicates which may be employed in, for example, above-mentioned areas as such or used as precursor for the preparation of respective tectosilicates or pillared silicates.

Thus, the present invention relates to a process for the preparation of an isomorphously substituted layered silicate comprising
(1) providing a mixture containing silica or a precursor thereof, at least one structure directing agent (SDA) allowing for the crystallization of the layered silicate, and water;
(2) heating the mixture obtained according to (1) under hydrothermal conditions to give a precursor suspension;
(3) adding at least one source of at least one element suitable for isomorphous substitution of at least a portion of the Si atoms in the layered silicate to the precursor suspension;
(4) heating the mixture obtained according to (3) under hydrothermal conditions to give the layered silicate.

Compared to the prior art processes, this process makes use of two separate crystallization steps. In a first step, the usual components used for the crystallization of all-silica silicates are admixed. However, this mixture (1) which is subjected to hydrothermal crystallization in step (2) does not yet contain the source for the at least one element to be incorporated in the desired layered silicate as isomorphously substituting element. Surprisingly, it was found that it is an advantage, especially in case the element suitable for isomorphous substitution is aluminium, to prepare, in a first crystallization step (2), a precursor suspension to which, in a subsequent step (3), the at least one source of the at least one element suitable for isomorphous substitution is added. Then, the resulting mixture comprised of the precursor suspension and the at least one source of the at least one element suitable for isomorphous substitution is subjected to a second hydrothermal crystallization step (4).

Any suitable compound can in principle be employed as silica or a silica precursor. The term "silica precursor" as used in this context refers to a compound which, under the chosen reaction conditions, allows for the formation of the silicate structure of the layered silicate. Tetraalkoxysilanes, such as tetramethoxysilane, tetraethoxysilane or tetrapropoxysilane, may be mentioned as precursor compound by way of example. In the process of the present invention, silica as such is particularly preferably employed. By way of example, such silica sources may be fumed, amorphous silica, silica sols such as Ludox or the like. It is also conceivable that a mixture of two or more silica sources or a mixture of two or more silica precursors or a mixture of at least one silica source and at least silica precursor is employed. Amorphous silica is especially preferred. Further, amorphous silica as the only silica source is preferred.

Therefore, the present invention also relates to the process as described above, wherein in (i), amorphous silica is employed.

Generally, it is in principle possible to employ any suitable amorphous silica. Amorphous silica having a specific surface (BET, Brunauer-Emmet-Teller; determined according to DIN 66131 by nitrogen adsorption at 77 K) in the range of from 10 to 400 m²/g, preferably in the range of from 10 to 100 m²/g, and particularly preferably in the range of from 10 to 50 m²/g is preferred. Further preferred ranges are from 50 to 100 m²/g or from 100 to 300 m²/g or from 300 to 400 m²/g.

As far as the structure directing agent is concerned, no specific limitations exist with the proviso that the structure directing agent must allow for the crystallization of the layered silicate.

According to the present invention, it is especially preferred to prepare layered silicates having RUB-39 or RUB-36 structure.

As to the structure directing agent allowing for the crystallization of a layered silicate having RUB-39 structure, dimethyldi-n-propylammonium compounds may be mentioned. According to the present invention, in addition to the dimethyldi-n-propylammonium compound, a base differing from this compound may be used. Examples of this base are ammonium hydroxide NH₄OH, alkali metal hydroxides or alkaline earth metal hydroxides, such as sodium hydroxide or potassium hydroxide, or mixtures of two or more of these compounds. In this case, the dimethyldi-n-propylammonium compound contains one or more suitable anions, for example halogen anions, such as fluoride or chloride or bromide or iodide. Preferably, the dimethyldi-n-propylammonium compound contains a base used according to (1) as an anion. Examples of basic anions in this context include, inter alia, the hydroxide ion. A particularly preferred basic anion is the hydroxide ion.

As to the structure directing agent allowing for the crystallization of a layered silicate having RUB-36 structure, diethyldimethylammonium and/or triethylmethylammonium compounds may be mentioned. According to the present invention, in addition to the diethyldimethylammonium and/or triethylmethylammonium compound, a base differing from this compound may be used. Examples of this base are ammonium hydroxide NH₄OH, alkali metal hydroxides or alkaline earth metal hydroxides, such as sodium hydroxide or potassium hydroxide, or mixtures of two or more of these compounds. In this case, the diethyldimethylammonium and/or triethylmethylammonium compound contains one or more suitable anions, for example halogen anions, such as fluoride or chloride or bromide or iodide. Preferably, the diethyldimethylammonium and/or triethylmethylammonium compound contains a base used according to (1) as an anion. Examples of basic anions in this context include, inter alia, the hydroxide ion. A particularly preferred basic anion is the hydroxide ion.

Therefore, the present invention also relates to the process as described above, wherein the at least one structure directing agent is dimethyldi-n-propylammonium hydroxide (DMDPAOH) or is selected from the group consisting of diethyldimethylammonium hydroxide, triethylmethylammonium hydroxide, and a mixture of diethyldimethylammonium hydroxide and triethylmethylammonium hydroxide, preferably DMDPAOH.

In addition to the SDA, the silica of the precursor thereof, and water, the mixture according to (1) preferably additionally contains at least one base, in addition to the hydroxide ion introduced via the preferred SDA. Among others, suitable hydroxides may be mentioned such as alkali metal hydroxides or alkaline earth metal hydroxides. In particular, sodium hydroxide was found to be a suitable additional basic component.

Therefore, the present invention also relates to the process as described above, wherein the mixture according to (1) additionally contains at least one base, preferably a hydroxide, more preferably sodium hydroxide.

Generally, the amount of additional base may be chosen according to respective desired reaction conditions. It is preferred that the mixture according to (1) SiO₂ or the precursor thereof, calculated as SiO₂, and the base, preferably NaOH, in a molar ratio SiO₂: base of 1 : (0.01-0.5), preferably of 1 : (0.02-0.1).

According to a still further preferred embodiment of the present invention, at least one suitable seeding material is contained in the mixture according to (1) as crystallization auxiliary. As seeding material, all compounds are conceivable resulting in the desired layered silicate after step (4).

Preferably, the layered silicate to be synthesized is added as seed material. While it is conceivable that also the isomorphously substituted layered silicate may be employed, it is especially preferable to add the all-silica layered silicate as seed material.

Therefore, the present invention also relates to the process as described above, wherein the mixture according to (1) additionally contains at least one suitable seed material, preferably seed crystals of the layered silicate, more preferably seed crystals of the all-silica layered silicate.

For the synthesis of the preferred layered silicate RUB-39, it is preferred to add, as seed material, the RUB-39 silicate and/or the tectosilicate RUB-41 obtained from this layered silicate by a process as described hereinunder. It is especially preferred to add, as seed material, the layered silicate RUB-39. Generally, it is possible to add the isomorphously substituted silicate as seeding material. It is preferred to add the all-silica silicate as seeding material. Therefore, it is especially preferred to add all-silica RUB-39 as seeding material.

For the synthesis of the preferred layered silicate RUB-36, it is preferred to add, as seed material, the RUB-36 silicate and/or the tectosilicate RUB-37 obtained from this layered silicate by a process as described hereinunder. It is especially preferred to add, as seed material, the layered silicate RUB-36. Generally, it is possible to add the isomorphously substituted silicate as seeding material. It is preferred to add the all-silica silicate as seeding material. Therefore, it is especially preferred to add all-silica RUB-36 as seeding material.

Typical concentrations of the seeding materials are in the range of from 0.001 to 5% by weight of seeding material, based on the silica or silica precursor, calculated as silica, present in the mixture according to (1). Preferably, the mixture obtained according to (1) contains SiO₂, or the silica precursor calculated as SiO₂, and the seed material in a weight ratio of 1 : (0.005-0.2), preferably of 1 : (0.01-0.1).

As far as above-mentioned molar ratios of silica or precursor thereof, SDA, and water are concerned, all ranges are conceivable which allow for the preparation of the silicates of the present invention. Ranges of the molar ratios of SiO₂: SDA: H₂O of 1 : (0.45-0.55) : (5-20), more preferably of 1 : (0.45-0.55): (6-18), and even more preferably 1: (0.45-0.55) : (7-16), especially preferably of 1 : (0.48-0.52): (8-14).

Even more preferably, the molar ratio of SO₂: SDA is preferably 1: (0.47-0.53), more preferable 1: (0.49-0.51).

In step (1) of the process of the present invention, a mixture is provided which is subjected to the first hydrothermal crystallization step (2). In general, it is possible to mix the individual starting materials in every conceivable order. Preferably, the SDA is first admixed with the seeding material. If, according to a preferred embodiment, an additional base such as sodium hydroxide is employed, it is further preferred to add the SDA to this base and add the seed material to this mixture. Preferably, the silica or the precursor thereof is added subsequently. Preferably, after each mixing step, the respectively resulting mixture may be stirred, generally for a period of time of, for example, from 5 minutes to 5 hours, preferably from 5 minutes to 2 hours.

The temperature during the preparation of the mixture according to (1) is preferably in the range of from 10 to 40 °C, more preferably in the range of from 15 to 35 °C, and particularly preferably in the range of from 20 to 30 °C.

Depending on the desired molar ratios of the starting materials, it is possible to either suitably remove or add a suitable amount of water. Removal of water may be achieved, for example, by carefully heating the mixture or using a rotary evaporator. The water is removed preferably at a temperature in the range of from 60 to 85 °C, more preferably of from 65 to 80°C, and particularly preferably of from 65 to 75 °C. If water is added or removed, it is preferred that the resulting mixture is stirred for 0.1 to 5 h, preferably from 0.2 to 1 h.

Generally, the stirring periods will also depend on the actual amount of starting materials. Thus, it is conceivable that for laboratory-scale manufacturing process, the stirring periods will be shorter than for an industrial-scale manufacturing process. The stirring periods described above preferably describe a laboratory-scale manufacturing process.

The resulting mixture is then subjected to the first hydrothermal crystallization in step (2). Preferably, the mixture is transferred in an autoclave. For adjusting the temperature of the crystallization to one or more desired temperatures, it is further preferred to use an autoclave which is equipped with heating and/or cooling means such as, e.g., internal and/or external heating and/or cooling means such as, e.g., a heating and/or cooling jacket. It is also possible to transfer the autoclave into an environment such as an oven, e.g. a circulating air oven, or the like which allows for maintaining a desired temperature in the synthesis mixture.

The synthesis mixture is preferably suitably stirred for the crystallization according to (2). It is also possible to rotate the reaction vessel in which the crystallization is carried out.

As far as the crystallization temperature is concerned, a temperature in the range of from 125 to 175 °C, more preferably from 130 to 160 °C, and even more preferably from 135 to 155 °C are used.

In particular, in case an RUB-39 silicate is produced, the temperature of the first crystallization step is in the range of from 145 to 155 °C, more preferably of from 146 to 154 °C.

In particular, in case an RUB-36 silicate is produced, the temperature of the first crystallization step is in the range of from 135 to 145 °C, more preferably of from 136 to 144 °C.

Also two or more different temperatures may be used during the crystallization in (2). In this context, it is possible to adjust the temperature to a certain value in the above-mentioned ranges, maintaining this temperature for a certain period of time, and then to increase or decrease the temperature to another value within above-mentioned ranges. Contrary or in addition to this stepwise adjustment of the temperature, the crystallization temperature may be gradually decreased or increased during hydrothermal crystallization. In general, the applied heating rates, either as far as heating the mixture according to (1) the crystallization temperature and/or as far as heating the mixture during (2) is/are concerned, can be suitably chosen. Preferably, the heating rates are in the range of from 0.1 °C/min to 20 °C/min, preferably from 0.3 °C/min to 15 °C/min and in particular from 0.5 °C/min to 10 °C/min.

As far as hydrothermal crystallization according to (2) is concerned, it is further preferred to maintain the crystallization temperature in step (2) for a period in the range of from 12 to 72 h, more preferably from 24 to 66 h and even more preferably from 36 to 60 h, such as, for example, of from 42 to 54 h.

The present invention also relates to the precursor suspension obtainable or obtained after step (2) by the process as described above.

According to the present invention, to the precursor suspension obtained in (2), at least one source at least one element suitable for isomorphous substitution of at least a portion of the Si atoms in the layered silicate is added in step (3). During the second hydrothermal crystallization of the process of the present invention according to (4), the at least one element is incorporated into the silicate framework at positions where Si would be placed in the respective all-silica silicate. The term "all-silica silicate" relates to silicates which, apart from unintentionally introduced impurities, consists of Si and O.

Generally, all elements, and sources thereof, respectively, may be employed which are suitable for such isomorphous substitution. Preferred suitable elements according to the present invention are selected from the group consisting of Al, B, Fe, Ti, Sn, Ga, Ge, Zr, V, Nb, and a mixture of two or more thereof. Aluminium is especially preferred. Due to the presence of the at least one source of the at least one suitable element, the silicate structure which is formed during hydrothermal crystallization in step (4) contains not only Si atoms, but also, as isomorphous substitution of Si atoms, at least one of the suitable elements.

If, for example, aluminum is incorporated as suitable element for isomorphous substitution, it is possible to use, for example, metallic aluminium or suitable aluminates, such as alkali metal aluminates, preferably sodium aluminate (NaAlO₂), and/or aluminium alcoholates, such as aluminium triisopropylate, also known as aluminium isopropoxide, as suitable sources.

Therefore, the present invention also relates to the process as described above, wherein the source of the at least one element suitable for isomorphous substitution is an aluminate, preferably sodium aluminate, or an aluminium alcoholate, preferably aluminium isopropoxide.

If, for example, boron is incorporated, it is possible to use, for example, free boric acid and/or borates and/or boric esters, such as triethyl borate, as suitable sources. If, for example, titanium is incorporated, it is possible to use, for example, titanium alcoholates, such as titanium ethanolates and/or titanium propylates and/or titanium butylates, as suitable sources. Therefore, the present invention also relates to the process as described above, wherein the source of Ti is a tetraalkyl-orthotitanate or a mixture of two or more tetraalkylorthotitanate, said tetraalkylorthotitanate preferably being selected from the group consisting of tetraethylorthotitanate, tetrapropylorthotitanate, tetrabutlyorthotitanate, and a mixture of two or three thereof. If, for example, tin is incorporated, it is possible to use, for example, tin chlorides and/or organometallic tin compounds, such as tin alcoholates, or chelates, such as tin acetylacetonates, in addition to the tetraalkylammonium compound and the silica and/or silica precursor as starting materials. If, for example, zirconium is incorporated, it is possible to use, for example, zirconium chloride and/or zirconium alcoholates in addition to the tetraalkylammonium compound and the silica and/or silica precursor as starting materials. If, for example, vanadium or germanium or niobium is incorporated, it is possible to use, for example, vanadium chloride or germanium chloride or niobium chloride in addition to the tetraalkylammonium compound and the silica and/or silica precursor as starting materials.

The amount of the at least one source of the at least one element, preferably Al, suitable for isomorphous substitution added in (3) can be chosen depending on the desired characteristics of the substituted layered silicate. Preferably, the molar ratios of SiO₂, or the silica precursor calculated as SiO₂, in the mixture according to (3) and the at least one element suitable for isomorphous substitution (1) are 1: (0.001- 0.5), more preferably 1: (0.005- 0.1), and especially preferably 1 : (0.01 - 0.05).

In step (3), the at least one source of the at least one suitable element may be added to the precursor suspension as such. It is also possible to add the at least one source of the at least one suitable element as aqueous mixture or solution. According to preferred embodiments of the present invention, the mixture according to (3) contains SiO₂ or the precursor thereof, calculated as SiO₂, the structure directing agent, and water in molar ratios SiO₂ : SDA : H₂O of 1 : (0.45-0.55) : (5-20), preferably 1 : (0.45-0.55) : (8-15). Therefore, depending on whether or not the at least one source of the at least one suitable element is added as aqueous mixture or solution, it may be necessary to adjust the water content of the mixture, either be adding water or by removing water. Such removal of water may be achieved, for example, by carefully heating the mixture or using a rotary evaporator. The water is removed preferably at a temperature in the range of from 60 to 85 °C, more preferably of from 65 to 80°C, and particularly preferably of from 65 to 75 °C. If water is added or removed, it is preferred that the resulting mixture is stirred for 0.1 to 5 h, preferably from 0.2 to 1 h. According to an especially preferred embodiment of the present, if water is removed, the autoclave is carefully heated to the temperatures as described above, thereby evaporating the water. Thus, it is not necessary to remove the precursor suspension obtained from (2) from the preferably used autoclave.

In step (3) of the process of the present invention, the mixture is provided which is subjected to the second hydrothermal crystallization step (4). Preferably, after having admixed the at least one source of the at least one suitable element with the precursor suspension, the respectively resulting mixture may be stirred, generally for a period of time of, for example, from 5 minutes to 5 hours, preferably from 5 minutes to 2 hours.

The resulting mixture is then subjected to the second hydrothermal crystallization in step (4). Preferably, the mixture is still in the autoclave already used in (2).

However, it is also conceivable to remove the mixture from the first autoclave and transfer it to another autoclave which may be better suited for the crystallization in step (4). For adjusting the temperature of the crystallization to one or more desired temperatures, it is further preferred to use an autoclave in the second step (4) which is equipped with heating and/or cooling means such as, e.g., internal and/or external heating and/or cooling means such as, e.g., a heating and/or cooling jacket. It is also possible to transfer the autoclave into an environment such as an oven, e.g. a circulating air oven, or the like which allows for maintaining a desired temperature in the synthesis mixture.

The synthesis mixture is preferably suitably stirred for the crystallization according to (4). It is also possible to rotate the reaction vessel in which the crystallization is carried out.

As far as the crystallization temperature is concerned, a temperature in the range of from 125 to 155 °C, more preferably from 130 to 150 °C, and even more preferably from 135 to 145 °C are used.

Also two or more different temperatures may be used during the crystallization in (4). In this context, it is possible to adjust the temperature to a certain value in the above-mentioned ranges, maintaining this temperature for a certain period of time, and then to increase or decrease the temperature to another value within above-mentioned ranges. Contrary or in addition to this stepwise adjustment of the temperature, the crystallization temperature may be gradually decreased or increased during hydrothermal crystallization. In general, the applied heating rates, either as far as heating the mixture according to (3) the crystallization temperature and/or as far as heating the mixture during (4) is/are concerned, can be suitably chosen. Preferably, the heating rates are in the range of from 0.1 °C/min to 20 °C/min, preferably from 0.3 °C/min to 15 °C/min and in particular from 0.5 °C/min to 10 °C/min.

As far as hydrothermal crystallization according to (4) is concerned, it is further preferred to maintain the crystallization temperature in step (4) for a period in the range of from 12 to 132 h, more preferably from 12 to 72 h, more preferably from 24 to 66 h and even more preferably from 36 to 60 h, such as, for example, of from 42 to 54 h.

The present invention also relates to the suspension obtainable or obtained after step (4) by the process as described above.

Thus, the present invention also relates to the isomorphously substituted layered silicate obtainable or obtained by the process consisting of steps (1) to (4), namely the as-synthesized isomorphously substituted layered silicate which still contains at least a portion of the SDA employed in (1).

According to the process of the present invention, the layered silicate contained in its mother liquor, obtained after hydrothermal crystallization in (4), is separated off in a suitable manner in at least one step from the suspension obtained from (4). This separation can be effected, for example, by means of filtration, ultrafiltration, diafiltration or centrifuging methods or, for example, spray drying and spray granulation methods. Separation by means of spray drying or filtration is preferred.

The separation can be followed by at least one washing step and/or at least one drying step, wherein it is possible to use identical or different washing agents or washing agent mixtures in at least two washing steps and to use identical or different drying temperatures in at least two drying steps.

If at least one washing step is conducted, it is preferred to wash the separated silicate until the pH of the washwater is in the range of from 6 to 8, preferably from 6.5 to 7.5, as determined via a standard glass electrode.

The drying temperatures here are preferably in the range of from room temperature to 180 °C, more preferably of from 55 to 165 °C, more preferably of from 65 to 150 °C, and particularly preferably in the range of from 75 to 125 °C.

Washing agents which may be used are, for example, water, alcohols, such as methanol, ethanol or propanol, or mixtures of two or more thereof. Examples of mixtures are mixtures of two or more alcohols, such as methanol and ethanol or methanol and propanol or ethanol and propanol or methanol and ethanol and propanol, or mixtures of water and at least one alcohol, such as water and methanol or water and ethanol or water and propanol or water and methanol and ethanol or water and methanol and propanol or water and ethanol and propanol or water and methanol and ethanol and propanol. Water or a mixture of water and at least one alcohol, preferably water and ethanol, is preferred, water being very particularly preferred as the only washing agent.

Therefore, the present invention also relates to the process as described above, additionally comprising
(5) separating the layered silicate from the suspension obtained according to (4);
(6) optionally washing, preferably to a pH of the washwater in the range of from 6 to 7, of the separated layered silicate, and/or
(7) optionally drying, preferably at a temperature in the range of from 75 to 125 °C, of the separated and optionally washed layered silicate.

Further, the present invention also relates to the isomorphously substituted layered silicate having the RUB-36 structure obtainable or obtained by the process consisting of steps (1), (2), (3), (4), and (5) and/or (6) and/or (7).

Further, the present invention relates to an isomorphously substituted RUB-36 silicate as such or as obtainable or obtained by the process as described above, wherein at least a portion of the Si atoms in the layered silicate is isomorphously substituted by at least one suitable element, wherein the at least one suitable element is preferably selected from the group consisting of Al, B, Fe, Ti, Sn, Ga, Ge, Zr, V, Nb and a mixture of two ore more thereof, more preferably Al, Preferably, the RUB-36 silicate of the present invention exhibits an atomic ratio of Si : suitable element, preferably Al, in the layered silicate which is in the range of 1 : (0.001-0.5), preferably of 1 : (0.005-0.1), more preferably of 1 : (0.01-0.05).

According to a further embodiment of the process of the present invention, the layered silicate obtained according to (4) is calcined according to (8) in at least one additional step. It is in principle possible to subject the suspension comprising the layered silicate, i.e. the mother liquor containing the layered silicate, directly to calcination. Preferably, the silicate is separated off from the suspension, as described above according to (5), prior to the calcination. Prior to the calcination, the silicate separated off from the suspension can be subjected to at least one washing step (6) as described above and/or at least one drying step (7) as described above.

The calcination according to (8) of the silicate obtained according to (4) and/or (5) and/or (6) and/or (7) is preferably effected at a temperature in the range of up to 700 °C to give an isomorphously substituted tectosilicate. More preferably, the calcination temperatures are in the range of from 300 to 700 °C, even more preferably of from 550 to 650 °C.

Thereby, according to a preferred embodiment of the process of the present invention, the heating of the layered silicate is carried out from room temperature to a temperature of up to 700 °C, the heating rate further preferably being in the range of from 0.1 to 20 °C/min, more preferably of from 0.2 to 10 °C/min, and particularly preferably in the range of from 0.5 to 5 °C/min.

According to a possible embodiment of the process of the present invention, the calcination is carried out stepwise at successive temperatures. The term "stepwise at successive temperatures" as used in the context of the present invention refers to a calcination in which the silicate to be calcined is heated to a certain temperature, is kept at this temperature for a certain time, and is heated from this temperature to at least one further temperature and is once again kept there for a certain time. If stepwise calcination is carried out, the silicate to be calcined is preferably kept at up to 4, more preferably at up to 3, particularly preferably at 2 temperatures.

The calcination can be effected in any suitable atmosphere, for example air, lean air, nitrogen, steam, synthetic air or carbon dioxide. The calcination is preferably effected under air.

The calcination can be carried out in any apparatus suitable for this purpose. The calcination is preferably effected in a rotating tube, in a belt calciner, in a muffle furnace, or in situ in an apparatus in which the silicate is subsequently used for the intended purpose, for example as a molecular sieve or for another application described below. A rotating tube and a belt calciner are particularly preferred here.

Accordingly, the present invention also relates to a process as described above, additionally comprising
(8) calcining the silicate obtained according to (4) or (5) or (6) or (7), preferably at a temperature in the range of from 300 to 700 °C, preferably in the range of from 550 to 650 °C.

The present invention accordingly also relates to an isomorphously substituted tectosilicate RUB-37, obtainable or obtained by the process as described above from the layered silicate RUB-36, said process comprising the calcination according to (8).

Further, the present invention relates to an isomorphously substituted RUB-37 silicate as such or as obtainable or obtained by the process as described above, wherein at least a portion of the Si atoms in the layered silicate is isomorphously substituted by at least one suitable element, wherein the at least one suitable element is preferably selected from the group consisting of Al, B, Fe, Ti, Sn, Ga, Ge, Zr, V, Nb and a mixture of two ore more thereof, more preferably Al, Preferably, the RUB-37 silicate of the present invention exhibits an atomic ratio of Si : suitable element, preferably Al, in the layered silicate which is in the range of 1 : (0.001-0.5), preferably of 1 : (0.005-0.1), more preferably of 1 : (0.01-0.05).

Also, the present invention relates to an RUB-37 silicate as such, wherein at least a portion of the Si atoms in the silicate is isomorphously substituted by at least one suitable element, wherein the at least one suitable element is preferably selected from the group consisting of Al, B, Fe, Ti, Sn, Ga, Ge, Zr, V, Nb and a mixture of two ore more thereof, more preferably from the group consisting of Ti, Al, B and a mixture of two or more thereof, even more preferably Ti or Al or B.

Preferably, the RUB-37 silicate of the present invention exhibits an atomic ratio of Si : suitable element, preferably Ti or B or Al in the layered silicate which is in the range of 1 : (0.0001- 0.1), more preferably of 1 : (0.001- 0.05), and even more preferably of 1 : (0.005 - 0.02).

In many technical applications, the user often desires to employ the isomorphously substituted layered silicate and/or the isomorphously substituted tectosilicate which has been processed to moldings, instead of the silicate material as such. Such moldings are necessary in particular in many industrial processes, in order, for example, to be able to expediently operate separations of substances from mixtures in, for example, tube reactors.

The present invention accordingly also relates to a molding comprising the isomorphously substituted layered silicate RUB-36 and/or the isomorphously substituted tectosilicate RUB-37, as described above.

In general, the molding may comprise all conceivable further compounds in addition to the isomorphously substituted layered silicate and/or the isomorphously substituted tectosilicate of the present invention, provided that it is ensured that the resulting molding is suitable for the desired application.

In the context of the present invention, it is preferred if at least one suitable binder material is used in the production of the molding. In the context of this preferred embodiment, more preferably a mixture of the isomorphously substituted layered silicate and/or the isomorphously substituted tectosilicate and the at least one binder is prepared. Suitable binders are in general all compounds which impart adhesion and/or cohesion between the particles of the the isomorphously substituted layered silicate and/or the isomorphously substituted tectosilicate which are to be bound, over and above the physisorption which may be present without a binder. Examples of such binders are metal oxides, such as SiO₂, Al₂O₃, TiO₂, ZrO₂ or MgO, or clays or mixtures of two or more of these compounds. As Al₂O₃ binders, clay minerals and naturally occurring or synthetic aluminas, for example alpha-, beta-, gamma-, delta-, eta-, kappa-, chi- or theta-alumina and the inorganic or organometallic precursor compounds thereof, such as gibbsite, bayerite, boehmite, pseudoboehmite or trialkoxyaluminates, such as aluminum triisopropylate are preferred in particular. Further preferred binders are amphiphilic compounds having a polar and a nonpolar moiety, and graphite. Further binders are, for example, clays, such as montmorillonites, kaolins, bentonites, halloysites, dickites, nacrites or anaxites. These binders can be used as such. In the context of the present invention, it is also possible to use compounds from which the binder is formed in at least one further step in the production of the moldings. Examples of such binder precursors are tetraalkoxysilanes, tetraalkoxytitanates, tetraalkoxyzirconates or a mixture of two or more different tetraalkoxysilanes or a mixture of two or more different tetraalkoxytitanates or a mixture of two or more different tetraalkoxyzirconates or a mixture of at least one tetraalkoxysilane and at least one tetraalkoxytitanate or of at least one tetraalkoxysilane and at least one tetraalkoxyzirconate or of at least one tetraalkoxytitanate and at least one tetraalkoxyzirconate or a mixture of at least one tetraalkoxysilane and at least one tetraalkoxytitanate and at least one tetraalkoxyzirconate. In the context of the present invention, binders which either completely or partly consist of SiO₂ or are a precursor of SiO₂, from which SiO₂ is formed in at least one further step in the production of the moldings are to be mentioned. In this context, both colloidal silica and "wet process" silica as well as "dry process" silica can be used. These are very particularly preferably amorphous silica, the size of the silica particles being, for example, in the range of from 5 to 100 nm and the surface of the silica particles being in the range of from 50 to 500 m²/g. Colloidal silica, preferably in the form of an alkaline and/or ammoniacal solution, more preferably in the form of an ammoniacal solution, is, for example, commercially available as, inter alia, Ludox®, Syton®, Nalco® or Snowtex®. "Wet process" silica is, for example, commercially available, inter alia, as Hi-Sil®, Ultrasil®, Vulcasil®, Santocel®, Valron-Estersil®, Tokusil® or Nipsil®. "Dry process" silica is, for example, commercially available, inter alia, as Aerosil®, Reolosil®, Cab-O-Sil®, Fransil® or ArcSilica®. The binders are preferably used in an amount which leads to the finally resulting moldings whose binder content is up to 80 % by weight, more preferably in the range of from 5 to 80 % by weight, more preferably in the range of from 10 to 70 % by weight, more preferably in the range of from 10 to 60 % by weight, more preferably in the range of from 15 to 50 % by weight, more preferably in the range of from 15 to 45 % by weight, particularly preferably in the range of from 15 to 40 % by weight, based in each case on the total weight of the finally resulting molding. The term "finally resulting molding" as used in the context of the present invention relates to a molding as obtained from the drying and calcining steps (IV) and/or (V), as described below, particularly preferably obtained from (V).

The mixture of binder or precursor of a binder and the the isomorphously substituted layered silicate and/or the isomorphously substituted tectosilicate material can be mixed with at least one further compound for further processing and for the formation of a plastic material. Here, inter alia, pore formers may preferably be mentioned. In the process of the present invention, all compounds which, with regard to the finished molding, provide a certain pore size and/or a certain pore size distribution and/or certain pore volumes can be used as pore formers. Preferably used pore formers in the process of the present invention are polymers which are dispersible, suspendable or emulsifiable in water or in aqueous solvent mixtures. Preferred polymers here are polymeric vinyl compounds, for example polyalkylene oxides, such as polyethylene oxides, polystyrene, polyacrylates, polymethacrylates, polyolefins, polyamides and polyesters, carbohydrates, such as cellulose or cellulose derivatives, for example methylcellulose, or sugars or natural fibers. Further suitable pore formers are, for example, pulp or graphite. If pore formers are used in the preparation of the mixture according to (I), the pore former content, preferably the polymer content of the mixture according to (I) is preferably in the range of from 5 to 90 % by weight, preferably in the range of from 15 to 75 % by weight, and particularly preferably in the range of from 25 to 55 % by weight, based in each case on the amount of RUB-36 and/or RUB-37 in the mixture according to (I). If desired for the pore size distribution to be achieved, a mixture of two or more pore formers may also be used. In a particularly preferred embodiment of the process of the present invention, as described below, the pore formers are removed in a step (V) by calcination to give the porous molding.

In the context of a likewise preferred embodiment of the present invention, at least one pasting agent is added in the preparation of the mixture according to (I). Pasting agents which may be used are all compounds suitable for this purpose. These are preferably organic, in particular hydrophilic polymers, for example cellulose, cellulose derivatives, such as methylcellulose, starch, such as potato starch, wallpaper paste, polyacrylates, polymethacrylates, polyvinyl alcohol, polyvinylpyrrolidone, polyisobutene or polytetrahydrofuran. Accordingly, particular compounds which also act as pore formers can be used as pasting agents. In a particularly preferred embodiment of the process of the present invention as described below, these pasting agents are removed in a step (V) by calcination to give the porous molding.

According to a further embodiment of the present invention, at least one acidic additive may added during the preparation of the mixture according to (I). Organic acidic compounds which can be removed in the preferred step (V), as described below, by calcination are very particularly preferred. Carboxylic acids, for example formic acid, oxalic acid and/or citric acid, are particularly preferred. It is also possible to use two or more of these acidic compounds.

The order of addition of the components of the mixture according to (I) which contains the the isomorphously substituted layered silicate and/or the isomorphously substituted tectosilicate is not critical. It is both possible first to add the at least one binder, then the at least one pore former and the at least one acidic compound and finally the at least one pasting agent and to interchange the sequence with regard to the at least one binder, the at least one pore former, the at least one acidic compound and the at least one pasting agent.

After the addition of the binder to the the isomorphously substituted layered silicate and/or the isomorphously substituted tectosilicate, to which, if appropriate, at least one of the compounds described above have already been added, the mixture according to (I) is, as a rule, homogenized for from 10 to 180 minutes. Inter alia, kneaders, edge mills or extruders are particularly preferably used for the homogenization. The mixture is preferably kneaded. On the industrial scale, treatment in an edge mill is preferably employed for the homogenization. The homogenization is carried out as a rule at temperatures in the range of from about 10 °C to the boiling point of the pasting agent and normal pressure or slightly superatmospheric pressure. Thereafter, if appropriate, at least one of the compounds described above can be added. The mixture thus obtained is homogenized, preferably kneaded, until an extrudable plastic material has formed.

According to a more preferred embodiment of the invention, the homogenized mixture is molded. In the context of the present invention, those processes in which the molding is effected by extrusion in conventional extruders, for example to give extrudates having a diameter of preferably from 1 to 10 mm, particularly preferably from 2 to 5 mm, are preferred for the shaping processes. Such extrusion apparatuses are described, for example, in Ullmann's Enzyklopädie der Technischen Chemie, 4th Edition, Vol. 2, page 295 et seq., 1972. In addition to the use of a screw-type extruder, a plunger-type extruder is also preferably used for the molding. In principle, however, all known and/or suitable kneading and molding apparatuses and processes may be used for the shaping. Examples of these are inter alia: briquetting, i.e. mechanical compression with or without addition of additional binder material; pelleting, i.e. co mpacting by circular and/or rotational movements; sintering, i.e. the material to be molded is subjected to a thermal treatment. The shape of the moldings produced according to the invention can be chosen as desired. In particular, inter alia spheres, oval shapes, cylinders or tablets are possible.

In the context of the present invention, step (III) is preferably followed by at least one drying step.

In the context of the present invention, the step (IV) is preferably followed by at least one calcination step. The calcination is carried out at temperatures in the range of, in general, from 300 to 700 °C, preferably from 400 to 600 °C. The calcination can be effected under any suitable gas atmosphere, air and/or lean air being preferred. Furthermore, the calcination is preferably carried out in a muffle furnace, a rotary kiln and/or a belt calcination oven. It is possible for the temperatures during a calcination step to remain constant or to be changed contiguously or discontinuously. If calcination is effected twice or more often, the calcination temperatures can be different or identical in the individual steps.

Accordingly, the present invention also relates to a process for the production of a molding as described above, comprising the steps
(I) preparing of a mixture containing the isomorphously substituted layered silicate and/or the isomorphously substituted tectosilicate as described above, and optionally at least one binder;
(II) kneading of the mixture;
(III) molding of the kneaded mixture to give at least one molding;
(IV) drying of the at least one molding;
(V) calcining of the at least one dried molding.

Before and/or after the drying and/or before and/or after the calcination, the at least one molding can, if appropriate, be treated with a concentrated or dilute Broenstedt acid or a mixture of two or more Broenstedt acids. Suitable acids are, for example, hydrochloric acid, sulfuric acid, phosphoric acid, nitric acid or carboxylic acids, dicarboxylic acids or oligo- or polycarboxylic acids, such as nitrilotriacetic acid, sulfosalicylic acid or ethylenediaminetetraacetic acid. If appropriate, this at least one treatment with at least one Broenstedt acid is followed by at least one drying step and/or at least one calcination step, which in each case is carried out under the conditions described above.

According to a further embodiment of the process of the present invention, the moldings obtained according to the invention can, for better hardening, be subjected to a water steam treatment, after which preferably drying is effected at least once again and/or calcination is effected at least once again. For example, after at least one drying step and at least one subsequent calcination step, the calcined molding is subjected to the steam treatment and is then dried at least once again and/or calcined at least once again.

The present invention moreover relates to the use of the isomorphously substituted layered silicate and/or the isomorphously substituted tectosilicate of the present invention, and/or of the moldings of the invention, as a molecular sieve, catalyst, catalyst support or binder thereof, as adsorbents, pigments, additives in detergents, an additive for building materials, for imparting thixotropic properties to coating pastes and finishes, and applications as external and internal lubricant, as flameproofing agent, auxiliary agent and filler in paper products, in bactericidal and/or fungicidal and/or herbicidal compositions, for ion exchange, for the production of ceramics, in polymers, in electrical, optical or electrooptical components and switching elements or sensors.

Preferably, the isomorphously substituted layered silicate and/or the isomorphously substituted tectosilicate of the present invention, and/or of the moldings of the invention may be used as a catalyst, a catalyst support or binder thereof, an adsorbent, for ion exchange, for the production of ceramics, or in polymers.

In particular, the isomorphously substituted tectosilicates of the present invention, further preferably the aluminium-substituted tectosilicates of the present invention, are used as a catalyst or catalyst component for amination reactions, preferably for the amination of methanol, or for MTO (methanol-to-olefin) conversion.

Further, the isomorphously substituted layered silicate may be used as starting materials for the manufacturing of pillard silicates.

The present invention is explained in more detail with reference to the examples and the figure described below. The examples of the present invention encompass laboratory-scale experiments as well as large-scale experiments. Thus, the broad applicability of the inventive process is shown.

### Description of the Figures

The powder X-ray diffraction patterns were recorded on a Siemens D-5000 with monochromatic Cu K alpha-1 radiation, a capillary sample holder being used in order to avoid a preferred orientation. The diffraction data were collected using a position-sensitive detector from Braun, in the range from 8 to 96 ° (2 Theta) and with a step width of 0.0678°. Indexing of the powder diagram was effected using the program Treor90, implemented in powder-X (Treor90 is a public domain program which is freely accessible via the URL http://www.ch.iucr.org/sincris-top/logiciel/). In the figures, the angle 2 Theta in ° is shown along the abscissa and the intensities are plotted along the ordinate.

### Examples

### Reference Example 1: Two-Step Synthesis of Al-RUB-39

### Step 1:

8.6 g of sodium hydroxide (NaOH) was dissolved in 229.3 g of water. This solution was then mixed with 653.4 g of aqueous solution of the template (SDA), dimethyldipropylammoniumhydroxide (39 wt %) under stirring at room temperature for 10 min. Then, 8.6 g of seed crystals of RUB-39 were added to this solution under stirring at room temperature for 20 min. Fumed silica (Aerosil) was added to this mixture in a stepwise fashion by consecutively introducing smaller portions, and a rather thick suspension was obtained. This suspension was stirred at room temperature for an additional 20 min. The resulting gel was poured into the autoclave. The molar composition of this synthesis mixture was SiO₂ : SDA : NaOH : H₂O = 1 : 0.5 : 0.0625 : 10. The first crystallization step was performed at 150 °C and the duration was 48 h. An opaque suspension with precipitation at the bottom was obtained after the first step.

### Step 2:

20.17 g of aluminum isopropoxide (aluminum triisopropylate) was directly introduced into the precursor obtained from the first step (1068 g). The mixture was stirred for 2 hours. The second crystallization step was performed at 140 °C and the duration was 48 h. An opaque suspension with precipitation at the bottom was obtained. The product was filtered (via centrifugation) and washed with water. The solid product was dried at 100 °C.

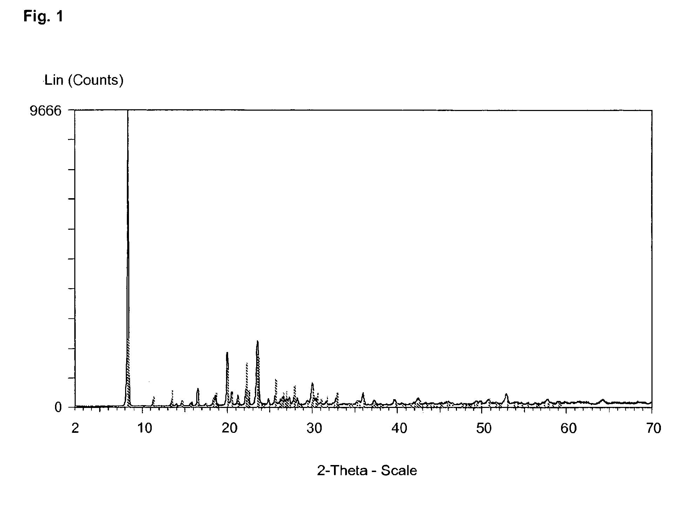

The XRD pattern of the obtained material is shown in Figure 1.

### Reference Example 2: Two-Step Synthesis of Al-RUB-39

### Step 1:

1.3 g of sodium hydroxide (NaOH) was dissolved in 35.4 g of water. This solution was then mixed with 100.9 g of aqueous solution of the template (SDA), dimethyldipropylammoniumhydroxide (39 wt %) under stirring at room temperature for 10 min. Then, 1.3 g of seed crystals of RUB-39 were added to this solution under stirring at room temperature for 20 min. Fumed silica (Aerosil) was added to this mixture in a stepwise fashion by consecutively introducing smaller portions, and a rather thick suspension was obtained. This suspension was stirred at room temperature for an additional 20 min. The resulting gel was poured into the autoclave. The molar composition of this synthesis mixture was SiO₂ : SDA : NaOH : H₂O = 1 : 0.5 : 0.0625 : 10. The first crystallization step was performed at 150 °C and the duration was 48 h. An opaque suspension with precipitation at the bottom was obtained after the first step.

### Step 2:

1.47 g of sodium aluminate (NaAlO₂) was dissolved in 53.7 g of water under stirring for 20 min. This solution was then mixed with the precursor obtained from the first step. The mixture was stirred for 30 min. The temperature was then increased to 70 °C and 53.7 g of water was evaporated from the synthesis mixture. The resulting gel was poured into the autoclave. The molar composition of this synthesis mixture was SiO₂ : NaAlO₂ SDA : NaOH : H₂O = 1 : 0.0333 : 0.5 : 0.0625 : 10. The second crystallization step was performed at 140 °C and the duration was 48 h. An opaque suspension with precipitation at the bottom was obtained. The product was filtered (via centrifugation) and washed with water. The solid product was dried at 100 °C.

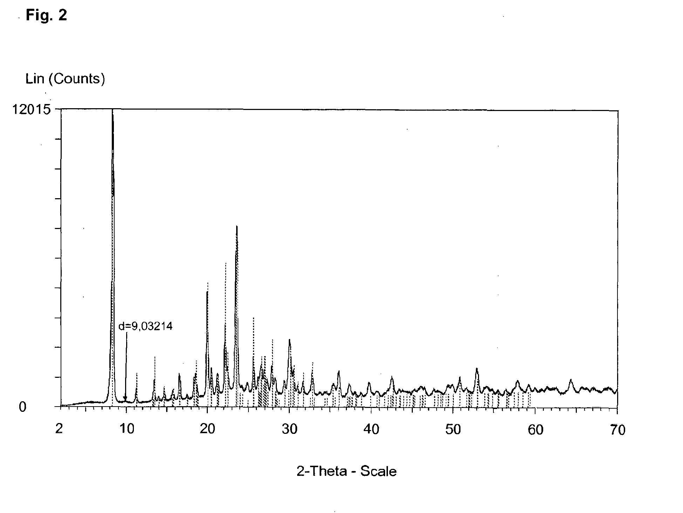

The XRD pattern of the obtained material is shown in Figure 2.

### Comparative Example: One-step synthesis of Al-RUB-39

The amounts of reagents used in this comparative examples were chosen to be about the same as the respective amounts used in example 3 hereinunder.

### Reagents:

1) NaOH 8.6g
2) H₂O 576.8g
3) dimethyldipropylammoniumhydroxide (39 wt.-%) aqueous solution 653.4 g
4) seed crystals (RUB-39) 8.6g
5) Aerosil 200 208.5g
6) NaAlO₂ 9.5g

NaOH was dissolved in 229.3 g of water followed by the addition of the dimethyldiethylammonium hydroxide solution and stirring of the resulting solution for 10 min. The seed crystals were then added and the solution was mixed for an additional 20 min. Aerosil was then added in portions while stirring and the mixture was then stirred for 20 min. A solution of NaAlO₂ dissolved in 347.5 g of water was then added to the mixture, which was then stirred for 30 min. 347.5 g of water were then removed from the mixture using a rotary evaporator, after which the concentrated mixture was transferred to a pressure digestion vessel and then heated therein under hydrothermal conditions at 140 °C for 120 h.

The resulting white suspension was centrifuged and the solid product was washed with distillated water and subsequently dried at 100 °C, thus yielding 2.65 g of product.

Contrary to the materials synthesized according to the present invention, the resulting product was found to be completely amorphous.

### Reference Example 3: Two-Step Synthesis of Al-RUB-39

### Step 1

### Reagents:

1) Aerosil 200 208.5g
2) dimethyldipropylammoniumhydroxide (39 wt.-%) aqueous solution 653.4g
3) seed crystals (RUB-39) 8.6g
4) H₂O 229.3g
5) NaOH 8.6g

NaOH was dissolved in water, to which dimethyldipropylammoniumhydroxide was then added and the resulting solution stirred for 10 min. The seed crystals were then added and the resulting mixture stirred for an additional 20 min. Aerosil was then added in portions while stirring and the resulting mixture then stirred for 1 h. The mixture was then transferred to a pressure digestion vessel and then heated therein under hydrothermal conditions at 150 °C for 48 h, yielding a brownish-white shimmering suspension.

### Step 2

### Reagents:

1) NaAlO₂ 9.3 g
2) H₂O 339.9 g

NaAlO₂ was dissolved in water and the resulting solution then stirred for 20 min. The solution was the added to the product of Step 1, after which the resulting mixture was stirred for 1 h. Subsequently, the mixture was transferred to a pressure digestion vessel and then heated therein under hydrothermal conditions at 140 °C for 48 h, yielding a grey-white shimmering suspension.

The reaction product was then centrifuged and the solid product washed with 2 liters of distillated water. The solid was then dried at 120 °C for 6 h, thus yielding 51.14 g of a white powder.

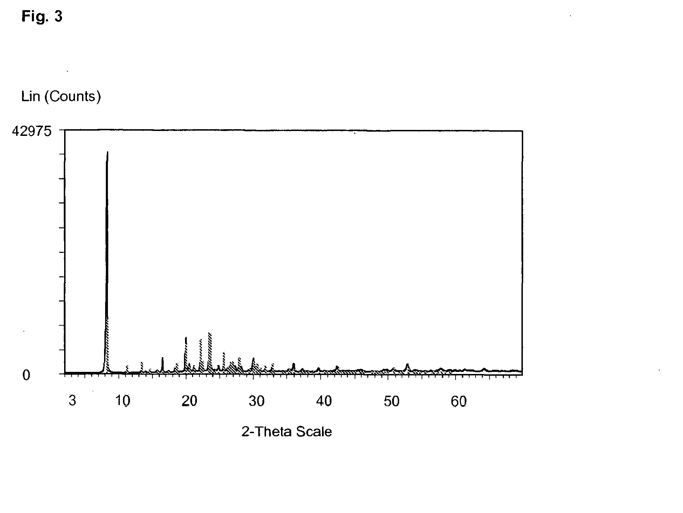

The XRD pattern of the obtained material is shown in Figure 3.

### Reference Example 4: Conversion of Al-RUB-39 to Al-RUB-41

36.838 g of the product obtained from Example 1 were heated to 600 °C at a rate of 1 °C/min and then held at that temperature for 10 h, thus yielding 29.507 g of Al-RUB-41.

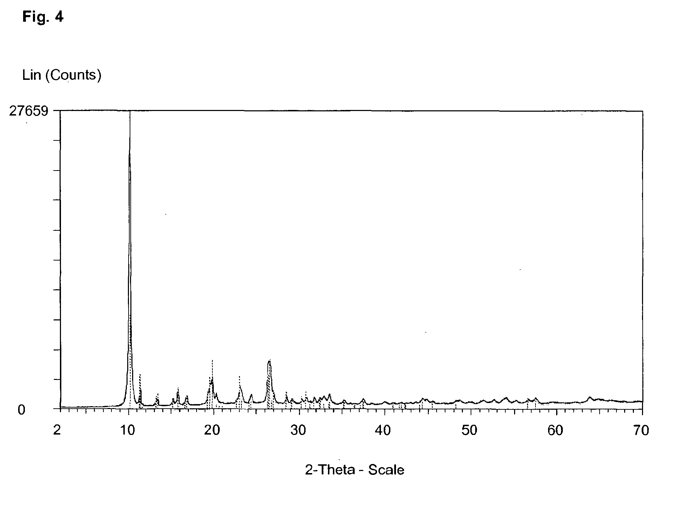

The XRD pattern of the obtained material is shown in Figure 4.

### Reference Example 5: Two-Step Synthesis of Al-RUB-39

### Step 1

### Reagents:

6) Aerosil 200 208.5g
7) dimethyldipropylammoniumhydroxide (39 wt.-%) aqueous solution 653.4g
8) seed crystals (RUB-39) 8.6g
9) H₂O 229.3g
10)NaOH 8.6g

NaOH was dissolved in water, to which dimethyldipropylammoniumhydroxide was then added and the resulting solution stirred for 10 min. The seed crystals were then added and the resulting mixture stirred for an additional 20 min. Aerosil was then added in portions while stirring and the resulting mixture then stirred for 1 h. The mixture was then transferred to a pressure digestion vessel and then heated therein under hydrothermal conditions at 140 °C for 48 h.

### Step 2

### Reagents:

1) aluminum triisopropylate 5.0 g

Aluminum triisopropylate was added to the product of Step 1, and the resulting mixture was stirred for 2 h. Subsequently, the mixture was transferred to a pressure digestion vessel and then heated therein under hydrothermal conditions at 140 °C for 48 h, yielding a yellow shimmering suspension.

The resulting suspension was separated by suction filtration, washed with 3 liters of distillated water, and subsequently dried at 120 °C for 19 h, yielding 35.55 g of a white powder.

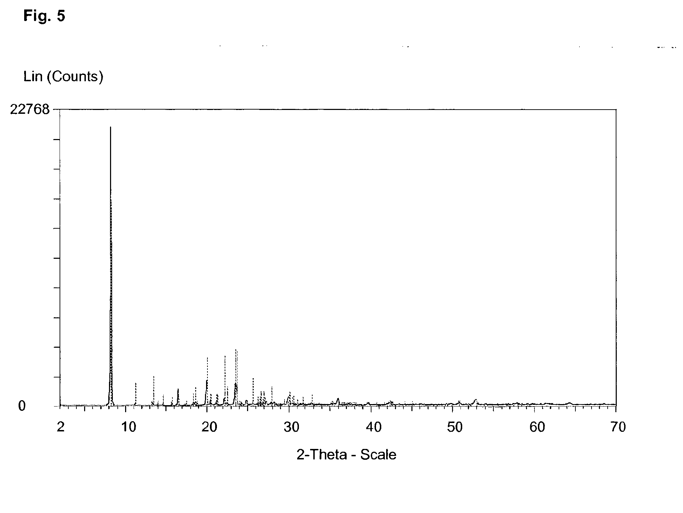

The XRD pattern of the obtained material is shown in Figure 5.

### Reference Example 6: Methanol Amination Experiment on Al-RUB-41

Catalysts were tested at 350°C or 400°C, with a N/C ratio of 2. Ammonia was fed as such; methanol was from a saturator with He. WHSV (weight hourly space velocity) was -0.6 g/g.h.

Al-RUB-39 from Example 4 was used in the experiments. It was first calcined to Al-RUB-41, then exchanged twice with NH₄Cl, calcined again and granulated.

A reference chabazite zeolite (obtained from BASF) was pretreated in the same way.

The catalysts were heated to 400°C under He before use.

### Results at 400°C:

| | H-Chabazite | H-Al-RUB-41 | thermodynamics at N/C = 2, 400°C |
|---|---|---|---|
| MeOH conversion (%) | 69 | 97.8 | |
| MMA sel. (%) | 56 | 50 | 30 |
| DMA sel. (%) | 36 | 38 | 36 |
| TMA sel. (%) | 8 | 12 | 34 |

Other non-amine products (likely olefins and dimethyl ether) are present, but only in amounts of a few %.

TMA formation is much less than it would be expected on a non-shape selective catalyst at N/C = 2. For example, up to 50 % are generally accepted for faujasite-type catalysts.

Conversion levels were observed in the first 2 hours of the run and were stable.

### Example 1: Two-Step Synthesis of Al-RUB-36

0.04 g of sodium hydroxide were added to 5.434 g of an aqueous diethyldimethylammonium hydroxide (21.9 wt.-%) solution while stirring. 1.2 g of fumed silica was then added to the mixture which was then stirred for 10 min. 0.036 g of RUB-36 was then added as seed crystals and the mixture was then stirred for 10 min. The resulting suspension was then transferred to a pressure digestion vessel and then heated therein under hydrothermal conditions at 140 °C for 48 h.

0.078 g of NaAlO₂ were dissolved in 2 ml of distilled water, and the solution was then added to the mixture resulting from the hydrothermal procedure. The mixture was then stirred for 10 min, and 2 g of water were then removed from the mixture using a rotary evaporator. The concentrated mixture was then transferred to a pressure digestion vessel and then heated therein under hydrothermal conditions at 40°C for 5 d (120 h).

The resulting solid product was then separated by suction filtration, washed with distillated water, and dried at 100 °C for 16 h, affording 0.558 g of product.

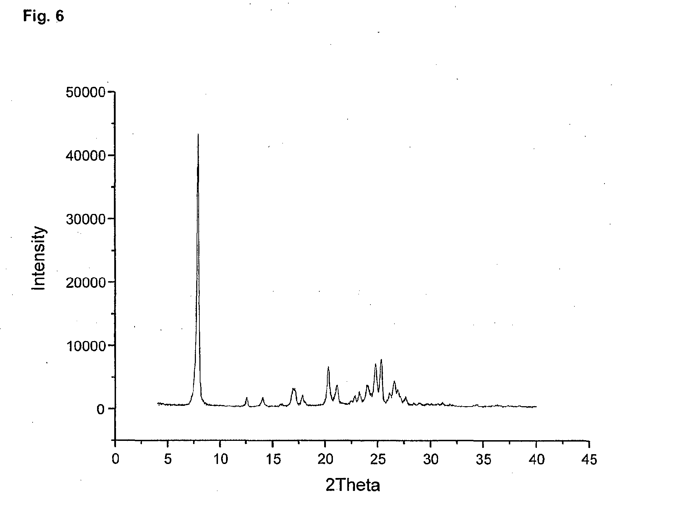

The XRD diffraction pattern is shown in Figure 6.

## Claims

1. A process for the preparation of an isomorphously substituted layered silicate comprising
(1) providing a mixture containing silica or a precursor thereof; at least one structure directing agent (SDA) allowing for the crystallization of the layered silicate, and water;
(2) heating the mixture obtained according to (1) under hydrothermal conditions to give a precursor suspension;
(3) adding at least one source of at least one element suitable for isomorphous substitution of at least a portion of the Si atoms in the layered silicate to the precursor suspension;
(4) heating the mixture obtained according to (3) under hydrothermal conditions to give the layered silicate.

2. The process of claim 1, wherein in (1), amorphous silica is employed.

3. The process of claim 1 or 2, wherein the at least one structure directing agent is dimethyldi-n-propylammonium hydroxide (DMDPAOH) or is selected from the group consisting of diethyldimethylammonium hydroxide, triethyl-methylammonium hydroxide, and a mixture of diethyldimethylammonium hydroxide and triethylmethylammonium hydroxide, preferably DMDPAOH.

4. The process of any of claims 1 to 3, wherein the mixture according to (1) additionally contains at least one base, preferably a hydroxide, more preferably sodium hydroxide.

5. The process of claim 4, wherein the mixture according to (1) contains SiO₂ or the precursor thereof, calculated as SiO₂, and the base, preferably NaOH, in a molar ratio SiO₂: base of 1 : (0.01-0.5), preferably of 1 : (0.02-0.1).

6. The process of any of claims 1 to 5, wherein the mixture according to (1) additionally contains at least one suitable seed material, preferably seed crystals of the layered silicate, more preferably seed crystals of the all-silica layered silicate.

7. The process of claim 6, wherein the mixture according to (1) contains SiO₂ or the precursor thereof, calculated as SiO₂, and the seed material in a weight ratio SiO₂ : seed material of 1 : (0.005-0.2), preferably of 1 : (0.01-0.1).

8. The process of any of claims 1 to 7, wherein the mixture according to (1) contains SiO₂ or the precursor thereof, calculated as SiO₂, the structure directing agent, and water in molar ratios SiO₂ : SDA : H₂O of 1 : (0.45-0.55) : (5-20), preferably 1 : (0.48-0.52) : (8-14).

9. The process of any of claims 1 to 8, wherein the mixture according to (1) is heated according to (2) for a period in the range of from 12 to 72 h, preferably from 36 to 60 h.

10. The process of any of claims 1 to 9, wherein the mixture is heated according to (2) to a temperature in the range of from 130 to 160 °C.

11. The process of any of claims 1 to 10, wherein the at least one element suitable for isomorphous substitution is selected from the group consisting of Al, B, Fe, Ti, Sn, Ga, Ge, Zr, V, Nb and a mixture of two or more thereof, preferably Al.

12. The process of any of claims 1 to 11, wherein the source of the at least one element suitable for isomorphous substitution is an aluminate, preferably sodium aluminate, or an aluminium alcoholate, preferably aluminium isopropoxide.

13. The process of any of claims 1 to 12, wherein the mixture according to (3) contains SiO₂ or the precursor thereof, calculated as SiO₂, and the at least one suitable element, preferably Al, in a molar ratio SiO₂ : element of 1 : (0.001-0.5), preferably of 1 : (0.005-0.1), more preferably of 1 : (0.01-0.05).

14. The process of any of claims 1 to 13, wherein the mixture according to (3) contains SiO₂ or the precursor thereof, calculated as SiO₂, the structure directing agent, and water in molar ratios SiO₂ : SDA : H₂O of 1 : (0.45-0.55) : (5-20), preferably 1 : (0.45-0.55): (8-15).

15. The process of any of claims 1 to 14, wherein the mixture according to (3) is heated according to (4) for a period in the range of from 12 to 132 h, preferably from 36 to 60 h.

16. The process of any of claims 1 to 15, wherein the mixture is heated according to (2) to a temperature in the range of from 135 to 145 °C.

17. The process of any of claims 1 to 16, additionally comprising
(5) separating the layered silicate from the suspension obtained according to (4);
(6) optionally washing, preferably to a pH of the washwater in the range of from 6 to 7, of the separated layered silicate, and/or
(7) optionally drying, preferably at a temperature in the range of from 75 to 125 °C, of the separated and optionally washed layered silicate.

18. The process of any of claims 1 to 17, additionally comprising
(8) calcining the silicate obtained according to (4) or (5) or (6) or (7), preferably at a temperature in the range of from 300 to 700 °C, preferably in the range of from 550 to 650 °C.

19. An isomorphously substituted layered silicate having the RUB-36 structure, obtainable by a process according to any of claims 1 to 17.

20. The layered silicate of claim 19, wherein the atomic ratio of Si : suitable element, preferably Al, in the layered silicate is in the range of 1 : (0.001-0.5), preferably of 1 : (0.005-0.1), more preferably of 1 : (0.01-0.05).

21. An isomorphously substituted tectosilicate having the RUB-37 structure, obtainable by a process according to claim 18.

22. The tectosilicate of claim 21, wherein the atomic ratio of Si : suitable element, preferably Al, in the tectosilicate is in the range of 1 : (0.001-0.5), preferably of 1 : (0.005-0.1), more preferably of 1 : (0.01-0.05).

23. A molding comprising the silicate of any of claims 19 to 22.

24. Use of silicate according to any one of claims 19 to 23 as a molecular sieve, catalyst, catalyst component, catalyst support or binder thereof, as an adsorbent, pigment, additive in detergents, additive for building materials, for imparting thixotropic properties to coating pastes and finishes, as external and internal lubricant, as a flameproofing agent, auxiliary agent and filler in paper products, in bactericidal and/or fungicidal and/or herbicidal compositions, for ion exchange, for the production of ceramics, in polymers, in electrical, optical or electrooptical components and switching elements or sensors.

25. The use of claim 24, preferably of the silicate according to claim 21 or 22, as catalyst or catalyst component for amination reactions, preferably for the amination of methanol, or for MTO (methanol-to-olefin) conversion.

## Patentansprüche

1. Verfahren zur Herstellung eines isomorph substituierten Schichtsilikats, umfassend
(1) Bereitstellen einer Mischung, die Siliciumdioxid oder eine Vorstufe davon, mindestens einen Strukturbildner (Structure Directing Agent, SDA), der die Kristallisation des Schichtsilikats erlaubt, und Wasser enthält;
(2) Erhitzen der gemäß (1) erhaltenen Mischung unter hydrothermalen Bedingungen zum Erhalt einer Vorstufensuspension;
(3) Zugeben mindestens einer Quelle mindestens eines für die isomorphe Substitution mindestens eines Teils der Si-Atome in dem Schichtsilikat geeigneten Elements zu der Vorstufensuspension;
(4) Erhitzen der gemäß (3) erhaltenen Mischung unter hydrothermalen Bedingungen zum Erhalt des Schichtsilikats.

2. Verfahren nach Anspruch 1, bei dem in (1) amorphes Siliciumdioxid eingesetzt wird.

3. Verfahren nach Anspruch 1 oder 2, bei dem der mindestens eine Strukturbildner Dimethyldi-n-propylammoniumhydroxid (DMDPAOH) ist oder aus der Gruppe bestehend aus Diethyldimethylammonium-hydroxid, Triethylmethylammoniumhydroxid und einer Mischung von Diethyldimethylammoniumhydroxid und Triethylmethylammoniumhydroxid ausgewählt wird und vorzugsweise DMDPAOH ist.

4. Verfahren nach einem der Ansprüche 1 bis 3, bei denen die Mischung gemäß (1) zusätzlich mindestens eine Base, vorzugsweise ein Hydroxid, weiter bevorzugt Natriumhydroxid, enthält.

5. Verfahren nach Anspruch 4, bei dem die Mischung gemäß (1) SiO₂ oder die Vorstufe davon, berechnet als SiO₂, und die Base, vorzugsweise NaOH, in einem Molverhältnis SiO₂ : Base von 1 : (0,01-0,5) und vorzugsweise 1 : (0,02-0,1) enthält.

6. Verfahren nach einem der Ansprüche 1 bis 5, wobei die Mischung gemäß (1) zusätzlich mindestens ein geeignetes Impfmaterial, vorzugsweise Impfkristalle des Schichtsilikats, weiter bevorzugt Impfkristalle des rein silikatischen Schichtsilikats, enthält.

7. Verfahren nach Anspruch 6, wobei die Mischung gemäß (1) SiO₂ oder die Vorstufe davon, berechnet als SiO₂, und das Impfmaterial in einem Gewichtsverhältnis SiO₂ : Impfmaterial von 1 : (0,005-0,2) und vorzugsweise 1 : (0,01-0,1) enthält.

8. Verfahren nach einem der Ansprüche 1 bis 7, wobei die Mischung gemäß (1) SiO₂ oder die Vorstufe davon, berechnet als SiO₂, den Strukturbildner und Wasser in Molverhältnissen SiO₂ : SDA : H₂O von 1 : (0,45-0,55) : (5-20) und vorzugsweise 1 : (0,48-0,52) : (8-14) enthält.

9. Verfahren nach einem der Ansprüche 1 bis 8, bei dem die Mischung gemäß (1) gemäß (2) über einen Zeitraum im Bereich von 12 bis 72 h und vorzugsweise von 36 bis 60 h erhitzt wird.

10. Verfahren nach einem der Ansprüche 1 bis 9, bei dem die Mischung gemäß (2) auf eine Temperatur im Bereich von 130 bis 160°C erhitzt wird.

11. Verfahren nach einem der Ansprüche 1 bis 10, bei dem das mindestens eine zur isomorphen Substitution geeignete Element aus der Gruppe bestehend aus Al, B, Fe, Ti, Sn, Ga, Ge, Zr, V, Nb und einer Mischung von zwei oder mehr davon ausgewählt wird und vorzugsweise Al ist.

12. Verfahren nach einem der Ansprüche 1 bis 11, bei dem es sich bei der Quelle des mindestens einen zur isomorphen Substitution geeigneten Elements um ein Aluminat, vorzugsweise Natriumaluminat, oder ein Aluminiumalkoholat, vorzugsweise Aluminiumisopropoxid, handelt.

13. Verfahren nach einem der Ansprüche 1 bis 12, wobei die Mischung gemäß (3) SiO₂ oder die Vorstufe davon, berechnet als SiO₂, und das mindestens eine geeignete Element, vorzugsweise Al, in einem Molverhältnis SiO₂ : Element von 1 : (0,001-0,5) vorzugsweise 1 : (0,005-1) und weiter bevorzugt 1 : (0,01-0,05) enthält.

14. Verfahren nach einem der Ansprüche 1 bis 13, wobei die Mischung gemäß (3) SiO₂ oder die Vorstufe davon, berechnet als SiO₂, den Strukturbildner und Wasser in Molverhältnissen SiO₂ : SDA : H₂O von 1 : (0,45-0,55) : (5-20) und vorzugsweise 1: (0,45-0,55) : (8-15) enthält.

15. Verfahren nach einem der Ansprüche 1 bis 14, bei dem die Mischung gemäß (3) gemäß (4) über einen Zeitraum im Bereich von 12 bis 132 h und vorzugsweise von 36 bis 60 h erhitzt wird.

16. Verfahren nach einem der Ansprüche 1 bis 15, bei dem die Mischung gemäß (2) auf eine Temperatur im Bereich von 135 bis 145°C erhitzt wird.

17. Verfahren nach einem der Ansprüche 1 bis 16, zusätzlich umfassend
(5) Abtrennen des Schichtsilikats aus der gemäß (4) erhaltenen Suspension;
(6) gegebenenfalls Waschen des abgetrennten Schichtsilikats, vorzugsweise bis zu einem pH-Wert des Waschwassers im Bereich von 6 bis 7, und/oder
(7) gegebenenfalls Trocknen des abgetrennten und gegebenenfalls gewaschenen Schichtsilikats, vorzugsweise bei einer Temperatur im Bereich von 75 bis 125°C.

18. Verfahren nach einem der Ansprüche 1 bis 17, zusätzlich umfassend
(8) Calcinieren des gemäß (4) oder (5) oder (6) oder (7) erhaltenen Silikats, vorzugsweise bei einer Temperatur im Bereich von 300 bis 700°C, vorzugsweise im Bereich von 550 bis 650°C.

19. Isomorph substituiertes Schichtsilikat mit der RUB-36-Struktur, das durch ein Verfahren gemäß einem der Ansprüche 1 bis 17 erhältlich ist.

20. Schichtsilikat nach Anspruch 19, wobei das Atomverhältnis Si : geeignetes Element, vorzugsweise Al, in dem Schichtsilikat im Bereich von 1 : (0,0001-0,5), vorzugsweise 1 : (0,005-0,1) und weiter bevorzugt 1 : (0,01-0,05) liegt.

21. Isomorph substituiertes Gerüstsilikat mit der RUB-37-Struktur, das durch ein Verfahren gemäß Anspruch 18 erhältlich ist.

22. Gerüstsilikat nach Anspruch 21, wobei das Atomverhältnis Si : geeignetes Element, vorzugsweise Al, in dem Gerüstsilikat im Bereich von 1 : (0,0001-0,5), vorzugsweise 1 : (0,005-0,1) und weiter bevorzugt 1 : (0,01-0,05) liegt.

23. Formkörper, umfassend das Silikat nach einem der Ansprüche 19 bis 22.

24. Verwendung von Silikat nach einem der Ansprüche 19 bis 23 als Molsieb, Katalysator, Katalysatorkomponente, Katalysatorträger oder Bindemittel dafür, als Adsorptionsmittel, Pigment, Zusatzstoff in Waschmitteln, Zusatz zu Baustoffen, zur Thixotropierung in Farbpasten und Lacken, als Gleit- und Schmierstoff, als Flammschutz, Hilfs- und Füllstoff in Papiererzeugnissen, in bakterizid und/oder fungizid und/oder herbizid wirksamen Zusammensetzungen, zum Ionenaustausch, zur Herstellung von Keramiken, in Polymeren, in elektrischen, optischen oder elektrooptischen Bauteilen und Schaltelementen oder Sensoren.

25. Verwendung nach Anspruch 24, vorzugsweise des Silikats nach Anspruch 21 oder 22, als Katalysator oder Katalysatorkomponente für Aminierungsreaktionen, vorzugsweise zur Aminierung von Methanol, oder für die MTO-Umwandlung (MTO = Methanol-To-Olefin).

## Revendications

1. Procédé de préparation d'un phyllosilicate à substitution isomorphe comprenant les étapes consistant à
(1) se procurer un mélange contenant de la silice ou un de ses précurseurs ; au moins un agent directeur de structure (SDA) permettant d'effectuer la cristallisation du phyllosilicate, et de l'eau ;
(2) chauffer le mélange obtenu en (1) dans des conditions hydrothermales pour donner une suspension de précurseur ;
(3) ajouter à la suspension de précurseur au moins une source d'au moins un élément convenable pour la substitution isomorphe d'au moins une partie des atomes de Si dans le phyllosilicate ;
(4) chauffer le mélange obtenu en (3) dans des conditions hydrothermales pour donner le phyllosilicate.

2. Procédé selon la revendication 1, dans lequel, en (1), de la silice amorphe est employée.

3. Procédé selon la revendication 1 ou 2, dans lequel le au moins un agent directeur de structure est l'hydroxyde de diméthyldi-n-propylammonium (DMDPAOH) ou est choisi dans le groupe constitué par l'hydroxyde de diéthyldiméthylammonium, l'hydroxyde de triéthylméthylammonium, et un mélange d'hydroxyde de diéthyldiméthyl-ammonium et d'hydroxyde de triéthylméthylammonium, de préférence DMDPAOH.

4. Procédé selon l'une quelconque des revendications 1 à 3, dans lequel le mélange selon (1) contient en plus au moins une base, de préférence un hydroxyde, de façon plus préférée de l'hydroxyde de sodium.

5. Procédé selon la revendication 4, dans lequel le mélange selon (1) contient SiO₂ ou son précurseur, calculé en SiO₂, et la base, de préférence NaOH, dans un rapport molaire SiO₂/base de 1/(0,01-0,5), de préférence de 1/ (0,02-0,1).

6. Procédé selon l'une quelconque des revendications 1 à 5, dans lequel le mélange selon (1) contient en plus au moins un matériau de germe convenable, de préférence des germes cristallins du phyllosilicate, de façon plus préférée des germes cristallins du phyllosilicate tout silice.

7. Procédé selon la revendication 6, dans lequel le mélange selon (1) contient SiO₂ ou son précurseur, calculé en SiO₂, et le matériau de germe dans un rapport pondéral SiO₂/matériau de germe de 1/(0,005-0,2), de préférence de 1/(0,01-0,1).

8. Procédé selon l'une quelconque des revendications 1 à 7, dans lequel le mélange selon (1) contient SiO₂ ou son précurseur, calculé en SiO₂, l'agent directeur de structure et l'eau dans des rapports molaires SiO₂/SDA/ H₂O de 1/(0,45-0,55)/(5-20), de préférence de 1/(0,48-0,52)/(8-14).

9. Procédé selon l'une quelconque des revendications 1 à 8, dans lequel le mélange selon (1) est chauffé selon (2) pendant un laps de temps dans la fourchette de 12 à 72 h, de préférence de 36 à 60 h.

10. Procédé selon l'une quelconque des revendications 1 à 9, dans lequel le mélange est chauffé selon (2) à une température dans la fourchette de 130 à 160 °C.

11. Procédé selon l'une quelconque des revendications 1 à 10, dans lequel le au moins un élément convenable pour la substitution isomorphe est choisi dans le groupe constitué par Al, B, Fe, Ti, Sn, Ga, Ge, Zr, V, Nb et un mélange de deux ou plus de deux d'entre eux, de préférence Al.

12. Procédé selon l'une quelconque des revendications 1 à 11, dans lequel la source du au moins un élément convenable pour la substitution isomorphe est un aluminate, de préférence de l'aluminate de sodium, ou un alcoolate d'aluminium, de préférence de l'isopropylate d'aluminium.

13. Procédé selon l'une quelconque des revendications 1 à 12, dans lequel le mélange selon (3) contient SiO₂ ou son précurseur, calculé en SiO₂, et le au moins un élément convenable, de préférence Al, dans un rapport molaire SiO₂/élément de 1/(0,001-0,5), de préférence de 1/(0,005-0,1), de façon plus préférée de 1/(0,01-0,05).

14. Procédé selon l'une quelconque des revendications 1 à 13, dans lequel le mélange selon (3) contient SiO₂ ou son précurseur, calculé en SiO₂, l'agent directeur de structure et l'eau dans des rapports molaires SiO₂/SDA/H₂O de 1/(0,45-0,55)/(5-20), de préférence de 1/(0,45-0,55)/(8-15).

15. Procédé selon l'une quelconque des revendications 1 à 14, dans lequel le mélange selon (3) est chauffé selon (4) pendant un laps de temps dans la fourchette de 12 à 132 h, de préférence de 36 à 60 h.

16. Procédé selon l'une quelconque des revendications 1 à 15, dans lequel le mélange est chauffé selon (2) à une température dans la fourchette de 135 à 145 °C.

17. Procédé selon l'une quelconque des revendications 1 à 16, comprenant en plus
(5) la séparation du phyllosilicate de la suspension obtenue en (4) ;
(6) éventuellement le lavage, de préférence à un pH de l'eau de lavage dans le domaine de 6 à 7, du phyllosilicate séparé, et/ou
(7) éventuellement le séchage, de préférence à une température dans la fourchette de 75 à 125 °C, du phyllosilicate séparé et éventuellement lavé.

18. Procédé selon l'une quelconque des revendications 1 à 17, comprenant en plus
(8) la calcination du silicate obtenu en (4) ou (5) ou en (6) ou (7), de préférence à une température dans la fourchette de 300 à 700 °C, de préférence dans la fourchette de 550 à 650 °C.

19. Phyllosilicate à substitution isomorphe ayant la structure RUB-36, susceptible d'être obtenu par un procédé selon l'une quelconque des revendications 1 à 17.

20. Phyllosilicate selon la revendication 19, dans lequel le rapport atomique Si/élément convenable, de préférence Al, dans le phyllosilicate est dans la fourchette de 1/(0,001-0,5), de préférence de 1/(0,005-0,1), de façon plus préférée de 1/(0,01-0,05).

21. Tectosilicate à substitution isomorphe ayant la structure RUB-37, susceptible d'être obtenu par un procédé selon la revendication 18.

22. Tectosilicate selon la revendication 21, dans lequel le rapport atomique Si/élément convenable, de préférence Al, dans le tectosilicate est dans la fourchette de 1/(0,001-0,5), de préférence de 1/(0,005-0,1), de façon plus préférée de 1/(0,01-0,05).

23. Moulage comprenant le silicate selon l'une quelconque des revendications 19 à 22.

24. Utilisation du silicate selon l'une quelconque des revendications 19 à 23 en tant que tamis moléculaire, catalyseur, composant de catalyseur, support de catalyseur ou liant pour catalyseur, en tant qu'adsorbant, pigment, additif dans les détergents, additif pour matériaux de construction, pour conférer des propriétés thixotropes à des pâtes et finitions de revêtement, en tant que lubrifiant externe et interne, en tant qu'agent ignifugeant, agent auxiliaire et charge dans les produits en papier, dans des compositions bactéricides et/ou fongicides et/ou herbicides, pour l'échange d'ions, pour la production de céramiques, dans des polymères, dans des composants électriques, optiques ou électrooptiques et des éléments de commutation ou des capteurs.

25. Utilisation selon la revendication 24, de préférence du silicate selon la revendication 21 ou 22, en tant que catalyseur ou composant de catalyseur pour des réactions d'amination, de préférence pour l'amination du méthanol, ou pour la conversion MTO (méthanol en oléfine).
